(19) Europäisches Patentamt
European Patent Office
Office européen des brevets

(11) Numéro de publication : **0 308 284 B1**

(12) # FASCICULE DE BREVET EUROPEEN

(45) Date de publication du fascicule du brevet : **11.03.92 Bulletin 92/11**

(51) Int. Cl.$^5$ : **C07D 211/94,** A61K 31/445

(21) Numéro de dépôt : **88402129.6**

(22) Date de dépôt : **19.08.88**

(54) **Dérivés de l'oxime du 1,2,5,6-tétrahydropyridine, leur procédé de préparation, leur application comme médicaments et les compositions les renfermant.**

(30) Priorité : **21.08.87 IT 2169187**

(43) Date de publication de la demande : **22.03.89 Bulletin 89/12**

(45) Mention de la délivrance du brevet : **11.03.92 Bulletin 92/11**

(84) Etats contractants désignés : **AT BE CH DE ES FR GB GR IT LI LU NL SE**

(56) Documents cités :
**FR-A- 1 258 847**
**CHEMICAL ABSTRACTS, vol. 92, no. 13, 31 mars 1980, résumé no. 110798y, Columbus, Ohio, US; I.A. KOZELLO et al.: "Improvement in the method of preparing arecoline based on acetaldehyde"**

(73) Titulaire : **ROUSSEL-UCLAF**
**35, Boulevard des Invalides**
**F-75007 Paris (FR)**

(72) Inventeur : **Toja, Emilio**
**Via Plezzo 80**
**I-20100 Milano (IT)**
Inventeur : **Bonetti, Carla**
**Via Beccalino**
**I-Fontanella (BG) (IT)**
Inventeur : **Barzaghi, Fernando**
**Via Monteverdi 21**
**I-20052 Monza (MI) (IT)**
Inventeur : **Galliani, Giulio**
**Via Silva 13**
**I-20052 Monza (MI) (IT)**

(74) Mandataire : **Tonnellier, Marie-José et al**
**111, route de Noisy B.P. no 9**
**F-93230 Romainville (FR)**

**EP 0 308 284 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés de l'oxime du 1,2,5,6-tétrahydropyridin-3-carboxaldéhyde, leur procédé de préparation et leur application comme médicaments.

L'invention a pour objet les composés répondant à la formule (I) :

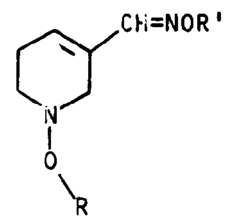

dans laquelle R représente soit un atome d'hydrogène, soit un radical alkyle linéaire, ramifié ou cyclique, saturé ou insaturé renfermant jusqu'à 8 atomes de carbone soit un radical

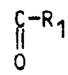

dans lequel R représente un radical alkyle linéaire ou ramifié, saturé ou insaturé renfermant jusqu'à 8 atomes de carbone, un radical alkoxy linéaire ou ramifié, saturé ou insaturé renfermant jusqu'à 8 atomes de carbone, ou un radical aryle renfermant jusqu'à 14 atomes de carbone,
soit un radical

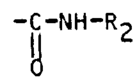

dans lequel R représente un radical alkyle linéaire ou ramifié, saturé ou insaturé renfermant jusqu'à 8 atomes de carbone, un radical aryle renfermant jusqu'à 14 atomes de carbone éventuellement substitué par un ou plusieurs substituants choisis parmi les atomes d'halogène, les radicaux alkyle renfermant jusqu'à 8 atomes de carbone et les radicaux alkoxy renfermant jusqu'à 8 atomes de carbone ou un radical aralkyle renfermant jusqu'à 18 atomes de carbone éventuellement substitué par un ou plusieurs substituants choisis parmi les atomes d'halogène, les radicaux alkyle renfermant jusqu'à 8 atomes de carbone et les radicaux alkoxy renfermant jusqu'à 8 atomes de carbone et R′ représente un radical alkyle linéaire ramifié ou cyclique, saturé ou insaturé renfermant jusqu'à 8 atomes de carbone ainsi que de leurs sels d'addition avec les acides organiques ou minéraux.

Parmi les sels d'addition avec les acides, on peut citer ceux formés avec les acides minéraux, tels que les acides chlorhydrique, bromhydrique, sulfurique ou phosphorique ou avec les acides organiques comme l'acide formique, acétique, propionique, benzoïque, maléique, fumarique, succinique, tartrique, citrique, oxalique, glyoxylique, aspartique, alcanesulfoniques, tels que les acides méthane ou éthane sulfoniques, arylsulfoniques tels que les acides benzène ou paratoluènesulfoniques.

Lorsque R, R, R ou R′ représente un radical alkyle saturé, linéaire ou ramifié, il s'agit de préférence d'un radical méthyle, éthyle, n-propyle, isopropyle, n-butyle, isobutyle, n-pentyle, n-hexyle, tert-butyle, tert-pentyle, néopentyle ou n-hexyle.

Lorsque R, R, R ou R′ représente un radical alkyle insaturé, il s'agit de préférence d'un radical éthylénique comme, par exemple, d'un radical vinyle, allyle, 1,1-diméthylallyle, but-2-ényle ou d'un radical acétylénique comme, par exemple, le radical éthynyle ou propynyle.

Lorsque R ou R′ représente un radical alkyle cyclique, il s'agit de préférence d'un radical cyclopropyle, cyclobutyle, cyclopentyle, cyclohexyle, cyclopropylméthyle, cyclobutylméthyle, cyclopentylméthyle ou cyclohexylméthyle.

Lorsque R ou R représente un radical aryle, il s'agit de préférence du radical phényle.

Lorsque R représente un radical aralkyle, il s'agit de préférence d'un radical benzyle ou phénéthyle.

Par atome d'halogène, on entend de préférence un atome de chlore ou de brome, par radical alkyle on entend de préférence un radical méthyle, éthyle, propyle linéaire ou ramifié, butyle linéaire ou ramifié et par

radical alcoxy, on entend de préférence un radical méthoxy, éthoxy, propoxy, butoxy.

Parmi les composés préférés de l'invention, on peut citer les composés dans lesquels R représente un atome d'hydrogène, un radical -COCH, un radical -CO-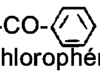 ou un radical -CONH-R dans lequel R représente un radical butyle, phényle, 4-méthoxyphényle, 4-chlorophényle ou 4-isopropylphényle.

On peut citer également les composés dans lesquels R′ représente un radical CH ou un radical CHC≡CH.

L'invention a naturellement plus particulièrement pour objet les composés de formule (I) dont la préparation est donnée ci-après dans la partie expérimentale.

Parmi ces composés, on peut citer les produits des exemples 1, 3, 4, 7, 10, 13, 15, 16 et tout particulièrement l'éther méthylique de (4-chlorophényl) amino carbonyloxy 1,2,5,6-tétrahydropyridin-3-carboxaldéhyde oxime ainsi que leurs sels d'addition avec les acides.

Les composés de l'invention présentent de très intéressantes propriétés pharmacologiques et notamment une activité cholinomimétique par voie orale importante et de longue durée d'action.

Il est bien connu que les troubles d'apprentissage et de la mémoire chez les personnes âgées sont surtout reliés à un déficit du système cholinergique central, en particulier dans la démence sénile et la maladie d'Alzheimer.

Il est donc évident que les produits ayant une action cholinergique centrale puissent être employés dans le traitement thérapeutique de ces maladies (Bartus, R.I. Science 217, 408, 1982).

Il a été démontré que l'arécoline injectée par voie intraveineuse a un effet positif sur des patients ayant un déficit de la mémoire (Sitaram N. et al. Science 201, 274, 1978) (Christie J. E. et al. Brit. J. Psychiatry 138, 46, 1981).

Une limitation à l'emploi thérapeutique de l'arécoline est reliée au fait que ce produit a une très faible activité par voir orale et une courte durée d'action.

Les produits, objet de l'invention, ont montré, après administration par voie orale, une activité cholinomimétique centrale jusqu'à 1000 fois supérieure à celle de l'arécoline et une plus longue durée d'action.

L'invention a donc pour objet les produits de l'invention en tant que médicaments utiles notamment dans le traitement de la maladie d'Alzheimer ou de la démence sénile et également dans le traitement des troubles de la mémoire.

Le médicament préféré est le composé de l'exemple 14.

La posologie usuelle est variable selon l'affection en cause, le sujet traité et la voie d'administration, elle peut être comprise entre 1 mg et 100 mg/jour, par exemple, entre 1 et 15 mg/jour en une ou plusieurs prises pour le produit de l'exemple 14 administré par voie orale.

La présente invention a également pour objet les compositions pharmaceutiques renfermant comme principe actif au moins un produit de formule (I).

Les compositions pharmaceutiques selon l'invention peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale au végétale, les dérivés paraffiniques, les glycols, les divers agents mouillants, dispersants ou émulsifiants, les conservateurs.

L'invention a également pour objet un procédé de préparation des composés de formule (I) caractérisé en ce que l'on soumet un composé de formule (II) :

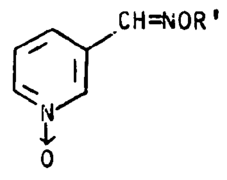

(II)

dans laquelle R′ a la signification indiquée précédemment à l'action d'un agent de réduction pour obtenir le composé de formule ($I_A$) correspondant au composé de formule (I) dans laquelle R représente un atome d'hydrogène

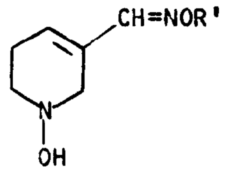

($I_A$)

que l'on soumet si désiré à l'action d'un agent d'estérification, d'éthérification, d'alkoxycarbonylation ou d'amidification pour obtenir le dérivé de formule (I) correspondant que l'on soumet si désiré, à l'action d'un acide pour en former le sel.

Dans un mode de réalisation préféré du procédé de l'invention:

- l'agent d'hydrogénation est l'hydroborure de sodium ;
- l'agent d'estérification est un halogénure d'acide ;
- l'agent d'éthérification est un dialkyl sulfate ;
- l'agent d'alkoxycarbonylation est un alkyl chloroformiate ;
- l'agent d'amidification est un isocyanate d'alkyle, d'aryle ou d'aralkyle.

Les produits de formule (II) utilisés comme produits de départ sont connus d'une façon générale. Certains sont décrits dans J. Heterocyclic Chem. 16, 1459 (1979).

Les exemples suivants illustrent l'invention sans toutefois la limiter.

**Exemple 1 : Chlorhydrate de l'éther méthylique du 1-hydroxy 1,2,5,6-tétrahydropyridin-3-carboxaldéhyde oxime.**

On dissout 1,2 g de N-oxyde pyridin-3-carboxaldéhyde 0-méthyloxime (J. Het. Chem. 16, 1459 (1979)) dans 40 cm3 de méthanol, refroidit à -10¤C, ajoute 0,9 g de borohydrure de sodium et agite à température ambiante pendant 1 heure. On concentre sous pression réduite, reprend avec de l'eau et extrait à l'éther. On évapore, chromatographie sur silice en éluant à l'acétate d'éthyle et obtient 0,87 g d'huile que l'on transforme en chlorhydrate. Après cristallisation dans un mélange d'isopropanol et d'éther, on obtient 0,9 g de chlorhydrate attendu. F = 146-147¤C.

| Analyse : | | | |
|---|---|---|---|
| Calculé : | C% 43,64 | H% 6,80 | N% 14,54 |
| Trouvé : | 43,85 | 6,76 | 14,52 |

**Exemple 2 : Chlorhydrate de l'éther méthylique du 1-méthoxy 1,2,5,6-tétrahydropyridin-3-carboxaldéhyde.**

A 3 g de (N-hydroxy 1,2,5,6-tétrahydropyridin-3-carboxaldéhyde 0-méthyloxime dissous dans 45 cm3 de tétrahydrofuranne refroidi à 0¤C, on ajoute 0,93 g d'hydrure de sodium à 55% dans l'huile. On refroidit à 0¤C pendant 15 minutes, ajoute 2,42 g de sulfate de diméthyle, maintient l'agitation à 0¤C pendant 15 minutes, puis à température ambiante pendant 1 heure. On évapore à sec, purifie le résidu par chromatographie sur silice (éluant : acétate d'éthyle puis chloroforme-méthanol 7-3 puis méthanol seul). On évapore à sec, reprend le résidu huileux au méthanol et salifie à l'aide d'acide chlorhydrique gazeux. Après cristallisation dans l'alcool isopropylique, on obtient 1,8 g de chlorhydrate attendu. F = 176¤C (décomp.).

| Analyse : | $C_8H_{14}N_2O_2$, HCl | | |
|---|---|---|---|
| Calculé : | C% 46,79 | H% 7,44 | N% 13,60 |
| Trouvé : | 46,49 | 7,32 | 13,56 |

Solubilité dans $H_2O$, HCl 2N et NaOH 2N.

Solubilité dans HO, HCl 2N et NaOH 2N.

**Exemple 3 : Chlorhydrate de l'éther méthylique du 1-acétoxy 1,2,5,6-tétrahydropyridin-3-carboxaldéhyde oxime.**

A 3,8 g de (1-hydroxy 1,2,5,6-tétrahydropyridin-3-carboxaldéhyde 0-méthyloxime dans 60 cm3 de tétrahydrofuranne, on ajoute 2,46 g de triéthylamine. On refroidit à 10¤C et ajoute 1,9 g de chlorure d'acétyle. Après 10 minutes à température ambiante, on filtre le chlorhydrate de triéthylamine, élimine le solvant par évaporation, purifie le résidu par chromatographie sur silice (éluant : acétate d'éthyle). Après cristallisation dans l'hexane, on obtient le produit attendu. F = 69-70¤C.

Analyse : $C_9H_{14}N_2O_3$

| | | | |
|---|---|---|---|
| Calculé : | C% 54,68 | H% 7,24 | N% 14,22 |
| Trouvé : | 54,53 | 7,12 | 14,13 |

Produit insoluble dans l'eau, dans la soude 2N, soluble dans l'acide chlorhydrique 2N.

**Exemple 4 : Chlorhydrate de l'éther propargylique du 1-hydroxy 1,2,5,6-tétrahydropyridin-3-carboxaldéhyde oxime.**

On dissout dans 100 cm3 de méthanol, 8,9 g de pyridine-3-carboxaldéhyde 0-2-propynyl oxime N-oxyde (préparé à partir de pyridin 3-carboxaldéhyde et d'0-2-propynyl hydroxylamine suivi d'une oxydation comme indiqué dans J. Het. Chem. 16, 1459 (1979) F = 104-105¤C), puis en maintenant la température inférieure à 5¤C, on ajoute 4,8 g de borohydrure de sodium. On agite à température ambiante pendant 2 heures, concentre sous pression réduite, reprend à l'eau et extrait à l'acétate d'éthyle. On sèche sur sulfate de soude et évapore sous pression réduite. On chromatographie sur silice (éluant : acétate d'éthyle) et obtient 4,5 g de produit huileux. On le dissout dans l'éther et salifie pac action d'un courant d'acide chlorhydrique gazeux. On obtient le chlorhydrate attendu, produit huileux très hydroscopique. Après cristallisation dans un mélange méthanol-/éther, on obtient 1,2 g de produit attendu sous forme de poudre blanche. F = 132-135¤C (décomp.) et environ 4 g d'huile.

Analyse :

| | | | |
|---|---|---|---|
| Calculé : | C% 49,89 | H% 6,05 | N% 12,93 |
| Trouvé : | 49,58 | 6,16 | 12,84 |

Soluble dans $H_2O$, HCl 2N et NaOH 2N.

Soluble dans HO, HCl 2N et NaOH 2N.

**Exemple 5 : Ether méthylique de 1-pivaloyloxy 1,2,5,6-tétrahydropyridin-3-carboxaldéhyde oxime.**

A un mélange de 0,8 g de l'éther méthylique de 1-hydroxy 1,2,5,6-tétrahydropyridin-3-carboxaldéhyde oxime obtenu à l'exemple 1, en solution dans 20 cm3 de tétrahydrofuranne et 0,52 g de triéthylamine, on ajoute à +10¤C, 0,63 g de chlorure de pivaloyle. On poursuit la réaction pendant 10 minutes à température ambiante, filtre le chlorhydrate de triéthylamine et évapore le solvant sous pression réduite. On chromatographie le résidu sur silice en éluant avec un mélange benzène-éther éthylique 1-1). On obtient 0,8 g de produit attendu (instable à la distillation).

Analyse : $C_{12}H_{20}N_2O_3$

| | | | |
|---|---|---|---|
| Calculé : | C% 59,98 | H% 8,39 | N% 11,66 |
| Trouvé : | 60,06 | 8,49 | 11,53 |

**Exemple 6 : Ether méthylique de 1-éthoxy carbonyloxy 1,2,5,6-tétrahydropyridin-3-carboxaldéhyde oxime.**

On opère comme à l'exemple 5 à partir de 1,4 g de produit de l'exemple 1, 0,91g de triéthylamine et 0,97 g de chloroformiate d'éthyle. On obtient 1,5 g de produit attendu.

Analyse : $C_{10}H_{16}N_2O_4$

| | | | |
|---|---|---|---|
| Calculé : | C% 52,62 | H% 7,06 | N% 12,67 |
| Trouvé : | 52,42 | 7,11 | 12,08 |

**Exemple 7 : Ether méthylique de 1-benzoyloxy 1,2,5,6-tétrahydropyridin-3-carboxaldéhyde oxime.**

A une solution de 1,56 g de produit obtenu à l'exemple 1 et 1,01 g de triéthylamine dans 30 cm3 de tétrahydrofuranne et refroidie à 10¤C, on ajoute 1,41 g de chlorure de benzoyle. On agite pendant 30 minutes à température ambiante. On reprend avec de l'acétate d'éthyle, lave à l'eau et évapore à sec. On purifie le résidu par chromatographie sur silice (éluant : toluène-acétate d'éthyle 8-2), on obtient 1,6 g de produit attendu cristallisé du cyclohexane. F = 74-76¤C.

Analyse : $C_{14}H_{16}N_2O_3$

| | | | |
|---|---|---|---|
| Calculé : | C% 64,6 | H% 6,20 | N% 10,76 |
| Trouvé : | 64,46 | 6,34 | 10,72 |

**Example 8 : Chlorhydrate de l'éther méthylique du 1-éthoxy 1,2,5,6-tétrahydropyridin-3-carboxaldéhyde oxime.**

A une solution de 3 g de produit de l'exemple 1 dans 50 cm3 de tétrahydrofuranne, on ajoute à 0¤C 0,93g d'hydrure de sodium à 85%. On adjoute ensuite à 0¤C 3 g d'iodure d'éthyle, laisse en attente 16 heures puis filtre l'insoluble et évapore à sec. On chromatographie sur silice (éluant : acétate d'éthyle puis méthanol-chloroforme 3-7). On chromatographie sur alumine en éluant avec méthanol-chloroforme (16-1). On obtient 1,8 g de résidu que l'on salifie avec du méthanol chlorhydrique et cristallise avec de l'éther éthylique. On obtient 1,05 g de produit attendu. F = 160-161¤C recristallisé d'un mélange isopropanol-éther éthylique.

Analyse : $C_9H_{16}N_2O_2$, HCl

| | | | |
|---|---|---|---|
| Calculé : | C% 48,98 | H% 7,76 | N% 12,69 |
| Trouvé : | 49,16 | 7,84 | 12,35 |

**Exemple 9 : Ether méthylique de 1-éthylamino carbonyloxy 1,2,5,6-tétrahydropyridin-3-carboxaldéhyde oxime.**

A une solution de 2 g de produit de l'exemple 1 dans 20 cm3 de toluène à 20¤C, on ajoute 1,82 g d'isocyanate d'éthyle, maintient pendant 40 minutes à température ambiante, évapore à secet obtient 1,5 g de produit attendu cristallisé de benzène.

Analyse : $C_{10}H_{17}N_3O_3$

| | | | |
|---|---|---|---|
| Calculé : | C% 52,85 | H% 7,54 | N% 18,49 |
| Trouvé : | 52,96 | 7,48 | 18,53 |

**Exemple 10 : Ether méthylique de 1-propyl aminocarbonyloxy 1,2,5,6-tétrahydropyridin-3-carboxaldéhyde oxime.**

On opère comme à l'exemple 9 en utilisant 2,1 g de produit de l'exemple 1 et 2,52 cm3 d'isocyanate de propyle. On obtient 2,85 g de produit attendu. F = 110-111¤C isolé du cyclohexane.

| | | | |
|---|---|---|---|
| Analyse : $C_{11}H_{19}N_3O_3$ | | | |
| Calculé : | C% 54,76 | H% 7,94 | N% 17,42 |
| Trouvé : | 54,57 | 7,87 | 17,19 |

**Exemple 11 : Ether méthylique de 1-isopropylaminocarbonyloxy 1,2,5,6-tétrahydropyridin-3-carboxaldéhyde oxime.**

A une solution de 1,2 g de produit obtenu comme à l'exemple 1 dans 20 cm3 de toluène, on ajoute 1,54 g d'isocyanate d'isopropyle. On agite pendant 4 heures à température ambiante, évapore à sec et chromatographie sur silice (éluant : acétate d'éthyle-toluène 7-3) cristallisé dans l'hexane. On obtient 1,3 g de produit attendu. F = 96-98¤C après cristallisation dans le cyclohexane.

| | | | |
|---|---|---|---|
| Analyse : $C_{11}H_{19}N_3O_3$ | | | |
| Calculé : | C% 54,76 | H% 7,94 | N% 17,42 |
| Trouvé : | 54,97 | 7,86 | 17,29 |

**Exemple 12 : Ether méthylique de 1-butylaminocarbonyloxy 1,2,5,6-tétrahydropyridin-3-carboxaldéhyde oxime.**

On opère comme à l'exemple 11 en utilisant 1,6 g de produit obtenu comme à l'exemple 1, 2,4 cm3 d'isocyanate de butyle. On obtient 1,5 g de produit attendu. F = 117-118¤C après cristallisation dans l'acétate d'éthyle.

| | | | |
|---|---|---|---|
| Analyse : $C_{12}H_{21}N_3O_3$ | | | |
| Calculé : | C% 56,45 | H% 8,29 | N% 16,46 |
| Trouvé : | 56,18 | 8,25 | 16,28 |

**Example 13 : Ether méthylique de 1-phénylaminocarbonyloxy 1,2,5,6-tétrahydropyridin-3-carboxaldéhyde oxime.**

On opère comme à l'exemple 9 en utilisant 1,25 g de produit obtenu comme à l'exemple 1 et 1,75 cm3 d'isocyanate de méthyle. On obtient 1,75 g de produit attendu. F = 76-78¤C.

| | | | |
|---|---|---|---|
| Analyse : $C_{14}H_{17}N_3O_3$ | | | |
| Calculé : | C% 61,08 | H% 6,22 | N% 15,26 |
| Trouvé : | 61,15 | 6,33 | 15,37 |

**Exemple 14 : Ether méthylique de 1-(4-chlorophénylaminocarbonyloxy) 1,2,5,6-tétrahydropyridin-3-carboxaldéhyde oxime.**

On opère comme à l'exemple 9 en utilisant 1,5 g de produit obtenu comme à l'exemple 1 et 3 g d'isocyanate de 4-chlorophényle. On obtient 2,84 g de produit attendu. F = 142-144¤C après cristallisation dans le cyclohexane.

Analyse : $C_{14}H_{16}ClN_3O_3$
Calculé : C% 54,29 H% 5,21 N% 13,57
Trouvé : 54,08 5,14 13,42

**Exemple 15 : Ether méthylique de 1-(4-isopropylphénylaminocarbonyloxy) 1,2,5,6-tétrahydropyridin-3-carboxaldéhyde oxime.**

On opère comme à l'exemple 9 en utilisant 1,56 g de produit obtenu comme à l'exemple 1 et 3,22 g de 4-isopropylphénylisocyanate. On obtient 2,8 g de produit attendu. F = 115-117¤C après cristallisation dans le cyclohexane.

Analyse : $C_{17}H_{23}N_3O_3$
Calculé : C% 64,33 H% 7,31 N% 13,24
Trouvé : 63,96 7,19 13,06

**Example 16 : Ether méthylique de 1-paraméthoxyphénylaminocarbonyloxy 1,2,5,6-tétrahydropyridin-3-carboxaldéhyde oxime.**

On opère comme à l'exemple 9 en utilisant 2,1 g de produit obtenu comme à l'exemple 1 et 3,5 cm3 d'isocyanate de p-méthoxyphényle. On obtient 2,95 g de produit attendu. F = 115-116¤C.

Analyse : $C_{15}H_{19}N_3O_4$
Calculé : C% 59,01 H% 6,27 N% 13,76
Trouvé : 59,14 6,22 13,58

En opérant comme à l'exemple 9 au départ des produits appropriés, on a préparé les produits des exemples 17 à 21.

**Exemple 17 : Ether méthylique de 3-chlorophénylaminocarbonyloxy 1,2,5,6-tétrahydropyridin-3-carboxaldéhyde oxime.**

F = 99-100¤C.

Analyse : $C_{14}H_{16}ClN_3O_3$ : 309,753
Calculé : C% 54,29 H% 5,21 N% 13,57
Trouvé : 54,07 5,17 13,45

**Exemple 18 : Ether méthylique de 3-méthoxyphénylaminocarbonyloxy 1,2,5,6-tétrahydropyridin-3-carboxaldéhyde oxime.**

F = 92-93¤C.

Analyse : $C_{15}H_{19}N_3O_4$ : 305,334
Calculé : C% 59,01 H% 6,27 N% 13,76
Trouvé : 59,13 6,31 13,64

**Exemple 19** : **Ether méthylique de 3,4-dichlorophénylaminocarbonyloxy 1,2,5,6-tétrahydropyridin-3-carboxaldéhyde oxime.**

F = 117-118¤C.

Analyse : $C_{14}H_{15}N_3O_3$

| | | | |
|---|---|---|---|
| Calculé : | C% 48,85 | H% 4,39 | N% 12,21 |
| Trouvé : | 49,03 | 4,55 | 12,09 |

**Exemple 20** : **Ether méthylique de 1-[[(1R) 1-phényl 1-éthyl] aminocarbonyloxy] 1,2,5,6-tétrahydropyridin-3-carboxaldéhyde oxime et isomère (1S) correspondant.**

Isomères (1R) et (1S) F = 115-116¤C.

Analyse : $C_{16}H_{21}N_3O_3$ : 303,362

| | | | |
|---|---|---|---|
| Calculé : | C% 63,35 | H% 6,98 | N% 13,85 |
| Trouvé : Isomère (1R) | 63,48 | 7,12 | 13,91 |
| Trouvé : Isomère (1S) | 63,59 | 6,99 | 13,77 |

**Exemple de compositions pharmaceutiques.**

a) On a préparé des comprimés répondant à la formule suivante :

- Produit de l'exemple 14 ........................................ 10 mg
- Excipient q.s. pour un comprimé terminé à ...................... 300 mg

(Détail de l'excipient : lactose, amidon de blé, amidon traité, amidon de riz, stéarate de magnésium, talc).

b) On a préparé des gélules répondant à la formule suivante :

- Produit de l'exemple 14 ........................................ 20 mg
- Excipient q.s. pour un gélule terminée à ...................... 300 mg

(Détail de l'excipient : talc, stéarate de magnésium, aérosil).

**ETUDE PHARMACOLOGIQUE**

**Toxicité aigüe.**

L'essai est réalisé sur des souris mâles (CD Charles Rivers) de 22 à 24 g, à jeun depuis 16 heures. On administre les produits par voie orale à la dose de 1000, 500, 250, 125, 62, 31 et 16 mg/kg. On note la mortalité pendant les 7 jours suivant le traitement.

| Exemple | $DL_{50}$ mg/kg |
|---|---|
| 1 | 60 |
| 3 | 100 |
| 4 | 80 |
| 6 | 125 |
| 7 | 90 |
| 10 | 125 |
| 13 | 250 |
| 14 | > 1000 |
| 15 | 350 |
| 16 | 60 |
| Arécoline HBr | 600 |

**Test de l'iléon isolé de cobaye.**

On prélève des fragments d'iléon de cobayes tués par décapitation. L'iléon isolé est placé dans 10 cm3 de solution de Tyrode à 37°C et aéré par un mélange d'oxygène (95%) et de gaz carbonique (5%). Les contractions dues aux produits sont enregistrées à l'aide d'un capteur relié à un polygraphe. Les produits à tester sont ajoutés aux concentrations comprises entre $1.10^{-s}$ et $1.10^{-x}$M/l.

Les produits présentant un effet contracturant sont testés vis à vis de l'atropine et de l'hexaméthonium pour établir si l'activité est de type "muscarinique" ou "nicotonique".

L'activité antagoniste éventuelle des produits est testés vis à vis de l'acétylcholine.

L'activité agoniste est exprimée en pD (logarithme négatif de la dose qui produit 50% de l'effet maximum).

L'activité antagoniste est exprimée en DE (dose réduisant de 50% la réponse maximale induite par l'acétylcholine).

| Exemple | $pD_2$ | $DE_{50}$ mg/kg |
|---|---|---|
| 1 | 5,38 | - |
| 3 | 4,90 | - |
| 4 | 5,24 | - |
| 6 | 4,94 | - |
| 7 | < 4 | > $1 \times 10^{-4}$ |
| 10 | < 4 | > $1 \times 10^{-4}$ |
| 13 | < 4 | > $1 \times 10^{-4}$ |
| 14 | < 4 | > $1 \times 10^{-4}$ |
| 15 | < 4 | > $1 \times 10^{-4}$ |
| 16 | < 4 | > $1 \times 10^{-4}$ |
| Arécoline | 6,48 | - |

**Activité diarrhéique.**

Le test est réalisé sur des souris mâles (CD Charles Rivers) pesant 25 à 30 g, à jeun depuis 6 heures. Le produit dissous à 5% dans du méthocel est administré par voie orale, au moyen d'une sonde oesophagienne.

Des animaux témoins ne reçoivent que l'excipient.

Après traitement, les animaux sont mis séparément dans des cages dont le fond est recouvert de papier buvard et sont mis en observation pendant 30, 60, 120 et 180 minutes.

Les feuilles de papier absorbant sont changées après chaque observation.

La consistance des fèces est évaluée selon la méthode de Randall et Baruth (Arch. Int. Pharmacodyn. 220, 94, 1976) en suivant l'échelle des valeurs suivantes.

0 : consistance ferme,

1 : fèces légèrement molles avec ou sans auréole humide,

2 : fèces légèrement molles avec présence d'un cercle humide bien défini,

3 : fèces molles avec présence d'un grand cercle humide,

4 : fèces sans consistance avec présence d'un très grand cercle humide.

Pour chaque produit, on a noté la dose qui provoque une diarrhée chez 50% des animaux selon la méthode de Miller et Tainter (Proc. Soc. Exp. Biol. Med., 57, 261, 1944).

| Exemple | $DE_{50}$ mg/kg |
| --- | --- |
| 1 | 0,5 |
| 3 | 0,5 |
| 4 | 0,9 |
| 6 | 0,6 |
| 7 | 1,5 |
| 10 | 20 |
| 13 | 5 |
| 14 | 10 |
| 15 | 20 |
| 16 | 6 |
| Arécoline | 35 |

**Activité hypothermique.**

Le test est réalisé sur des souris mâles (CD Charles Rivers) pesant 25 à 30 g, à jeun depuis 6 heures.

La température du corps est notée au moyen d'un thermocouple placé dans le rectum à environ 1,5 cm, et relié à un enregistreur de température électrique.

Les produits sont administrés par voie orale ou sous-cutanée et les températures sont notées à l'instant 0 et 30 minutes, 1 heure, 2 heures et 2 heures et demie après traitement.

On évalue le degré d'hypothermie comme la différence entre les animaux traités et les témoins et on détermine la dose nécessaire pour réduire de 1¤C la température du corps.

| Exemple | Dose efficace (-1¤C) en mg/kg V.O | V.SC |
|---|---|---|
| 1 | 0,19 | 0,20 |
| 3 | 0,22 | 0,20 |
| 4 | 0,48 | 0,49 |
| 6 | 0,26 | 0,24 |
| 7 | 0,42 | 0,21 |
| 10 | 1,4 | 1,3 |
| 13 | 0,74 | 0,43 |
| 14 | 1,8 | 1,8 |
| 15 | 1,9 | 2,4 |
| 16 | 0,88 | 0,78 |
| Arécoline, HBr | 194 | 3 |

**Variation de la température corporelle.**

On détermine la durée d'action des produits en utilisant des doses capables de réduire la température de 1¤ à 1,5¤C.

Variations de la température du corps (°C)

| Exemple | dose mg/kg | administration | temps en minute de traitement 0 | 30 | 60 | 120 | 180 |
|---|---|---|---|---|---|---|---|
| 1 | 0,25 | os | + 0,1 | - 1,4** | - 1,2** | - 0,1 | - 0,1 |
|  | 0,25 | sc | ± 0 | - 1,4** | - 1,1** | - 0,1 | - 0,1 |
| 3 | 0,25 | os | + 0,2 | - 1,2** | - 0,7** | + 0,1 | + 0,1 |
|  | 0,25 | sc | + 0,1 | - 1,2** | - 0,8** | + 0,1 | + 0,1 |
| 4 | 0,75 | os | ± 0 | - 1,6** | - 1,0** | ± 0 | - 0,1 |
|  | 0,75 | sc | ± 0 | - 1,4** | - 1,1** | ± 0,1 | ± 0 |
| 6 | 0,35 | os | ± 0 | - 1,3** | - 1,1** | - 0,1 | ± 0 |
|  | 0,35 | sc | ± 0 | - 1,4** | - 1,1** | - 0,1 | ± 0,1 |
| 7 | 0,5 | os | - 0,1 | - 1,1** | - 0,9** | ± 0 | + 0,1 |
|  | 0,25 | sc | - 0,1 | - 1,2** | - 0,9** | ± 0 | + 0,1 |
| 10 | 1,56 | os | - 0,2 | - 1,2** | - 1,0** | - 0,1 | + 0,1 |
|  | 1,56 | sc | ± 0 | - 1,2** | - 1,1** | - 0,1 | + 0,1 |
| 13 | 1 | os | + 0,2 | - 1,4** | - 1,1** | - 0,1 | ± 0 |
|  | 0,5 | sc | + 0,1 | - 1,2** | - 1,2** | - 0,1 | ± 0,1 |
| 14 | 2,5 | os | + 0,1 | - 1,3** | - 1,5** | - 0,3** | + 0,1 |
|  | 2,5 | sc | ± 0 | - 0,4** | - 1,5** | - 1,3** | - 0,2 |
| 15 | 2 | os | + 0,2 | - 0,8** | - 1,0** | ± 0 | + 0,1 |
|  | 4 | sc | + 0,2 | - 0,5** | - 1,5** | ± 1,5** | - 0,3 |
| 16 | 1 | os | + 0,1 | - 1,1** | - 1,1** | - 0,1 | ± 0 |
|  | 1 | sc | ± 0 | - 1,2** | - 1,5** | - 0,3* | ± 0,1 |
| Arécoline,HBr | 200 | os | + 0,1 | - 1,1** | - 1,0** | - 0,2 | - 0,1 |
|  | 3,5 | sc | - 0,1 | - 1,5** | - 0,1 | + 0,2 | + 0,2 |

** Valeurs différentes des témoins d'une manière significative (p< 0,01); (* p<0,5)

## Revendications

### Revendications pour les Etats contractants suivants : AT, BE, CH, DE, FR, GB, IT, LI, LU, NL, SE

1. Les composés répondant à la formule (I) :

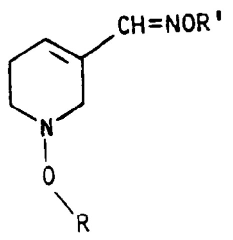

dans laquelle R représente soit un atome d'hydrogène, soit un radical alkyle linéaire, ramifié ou cyclique, saturé ou insaturé renfermant jusqu'à 8 atomes de carbone soit un radical

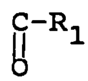

dans lequel R représente un radical alkyle linéaire ou ramifié, saturé ou insaturé renfermant jusqu'à 8 atomes de carbone, un radical alkoxy linéaire ou ramifié, saturé ou insaturé renfermant jusqu'à 8 atomes de carbone, ou un radical aryle renfermant jusqu'à 14 atomes de carbone, soit un radical

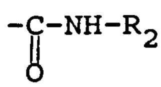

dans lequel R représente un radical linéaire ou ramifié, saturé ou insaturé renfermant jusqu'à 8 atomes de carbone, un radical aryle renfermant jusqu'à 14 atomes de carbone éventuellement substitué par un ou plusieurs substituants choisis parmi les atomes d'halogène, les radicaux alkyle renfermant jusqu'à 8 atomes de carbone et les radicaux alkoxy renfermant jusqu'à 8 atomes de carbone ou un radical aralkyle renfermant jusqu'à 18 atomes de carbone éventuellement substitué par un ou plusieurs substituants choisis parmi les atomes d'halogène, les radicaux alkyle renfermant jusqu'à 8 atomes de carbone et les radicaux alkoxy renfermant jusqu'à 8 atomes de carbone et R′ représente un radical alkyle linéaire ramifié ou cyclique, saturé ou insaturé renfermant jusqu'à 8 atomes carbone, ainsi que de leurs sels d'addition avec les acides organiques ou minéraux.

2. Les composés de formule (I) tels que définis à la revendication 1 dans lesquels R représente un atome d'hydrogène ainsi que leurs sels d'addition avec les acides organiques ou minéraux.

3. Les composés de formule (I) tels que définis à la revendication 1 dans lesquels R représente un radical -COCH ainsi que leurs sels d'addition avec les acides organiques ou minéraux.

4. Les composés de formule (I) tels que définis à la revendication 1 dans lesquels R représente un radical -CO-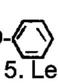 ainsi que leurs sels d'addition avec les acides organiques ou minéraux.

5. Les composés de formule (I) tels que définis à la revendication 1 dans lesquels R représente un radical -CONH-R dans lequel R représente un radical butyle, un radical phényle, un radical 4-méthoxyphényle, un radical 4-chlorophényle ou un radical 4-isopropylphényle.

6. Les composés de formule (I) tels que définis à l'une quelconque des revendications 1 à 5 dans lesquels R′ représente un radical -CH ainsi que leurs sels d'addition avec les acides organiques ou minéraux.

7. Les composés de formule (I) tels que définis à l'une quelconque des revendications 1 à 5 dans lesquels R′ représente un radical -CHC=CH ainsi que leurs sels d'addition avec les acides organiques ou minéraux.

8 - Les composés de formule (I) tels que définis à la revendication 1 dont le nom suit :

– l'éther méthylique de (4-chlorophényl) aminocarbonyloxy 1,2,5,6-tétrahydropyridin-3-carboxaldéhyde oxime ainsi que ses sels d'addition avec les acides.

9. A titre de médicaments, les composés de formule (I) tels que définis à l'une quelconque des revendications 1 à 7, ainsi que leurs sels d'addition avec les acides pharmaceutiquement acceptables.

10. A titre de médicaments, les composés de formule (I) tels que définis à la revendication 8, ainsi que leurs sels d'addition avec les acides pharmaceutiquement acceptables.

11. Les compositions pharmaceutiques renfermant à titre de principe actif au moins l'un des médicaments définis à la revendication 9 ou 10.

12. Procédé de préparation des composés de formule (I) tels que définis à l'une quelconque des revendications 1 à 8, caractérisé en ce que l'on soumet un composé de formule (II) :

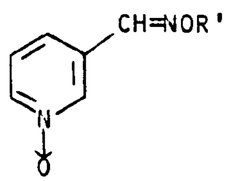

(II)

dans laquelle R' a la signification indiquée précédemment à l'action d'un agent de réduction pour obtenir le composé de formule ($I_A$) correspondant au produit de formule (I) dans laquelle R représente un atome d'hydrogène

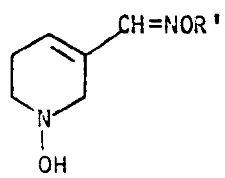

($I_A$)

que l'on soumet si désiré à l'action d'un agent d'estérification, d'éthérification, d'alkoxycarbonylation nu d'amidification pour obtenir le dérivé de formule (I) correspondant que l'on soumet si désiré, à l'action d'un acide pour en former le sel.

**Revendications pour l'Etat contractant suivant : ES**

1. Procédé pour préparer les composés répondant à la formule (I) :

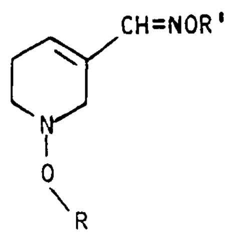

dans laquelle R représente soit un atome d'hydrogène, soit un radical alkyle linéaire, ramifié ou cyclique, saturé ou insaturé renfermant jusqu'à 8 atomes de carbone soit un radical

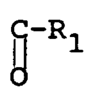

dans lequel R représente un radical alkyle linéaire ou ramifié, saturé ou insaturé renfermant jusqu'à 8 atomes de carbone, un radical alkoxy linéaire ou ramifié, saturé ou insaturé renfermant jusqu'à 8 atomes de carbone, ou un radical aryle renfermant jusqu'à 14 atomes de carbone, soit un radical

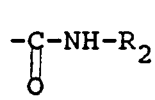

dans lequel R représente un radical alkyle linéaire ou ramifié, saturé ou insaturé renfermant jusqu'à 8 atomes de carbone, un radical aryle renfermant jusqu'à 14 atomes de carbone éventuellement substitué par un ou plusieurs substituants choisis parmi les atomes d'halogène, les radicaux alkyle renfermant jusqu'à 8 atomes de carbone et les radicaux alkoxy renfermant jusqu'à 8 atomes de carbone ou un radical aralkyle renfermant jusqu'à 18 atomes de carbone éventuellement substitué par un ou plusieurs substituants choisis parmi les atomes d'halogène, les radicaux alkyle renfermant jusqu'à 8 atomes de carbone et les radicaux alkoxy renfermant jusqu'à 8 atomes de carbone et R′ représente un radical alkyle linéaire ramifié ou cyclique, saturé ou insaturé renfermant jusqu'à 8 atomes carbone, ainsi que de leurs sels d'addition avec les acides organiques ou minéraux, caractérisé en ce que l'on soumet un composé de formule (II) :

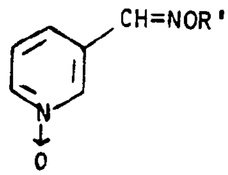

(II)

dans laquelle R′ a la signification indiquée précédemment à l'action d'un agent de réduction pour obtenir le composé de formule ($I_A$) correspondant au produit de formule (I) dans laquelle R représente un atome d'hydrogène

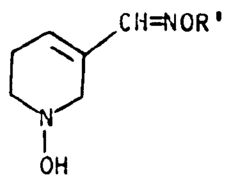

($I_A$)

que l'on soumet si désiré à l'action d'un agent d'estérification, d'éthérification, d'alkoxycarbonylation ou d'amidification pour obtenir le dérivé de formule (I) correspondant que l'on soumet si désiré, à l'action d'un acide pour en former le sel.

2. Procédé selon la revendication 1, caractérisé en ce que l'on utilise au départ, un composé de formule (II) dans laquelle R′ représente un radical -CH.

3. Procédé selon la revendication 1, caractérisé en ce que l'on utilise au départ , un composé de formule (II) dans laquelle R′ représente un radical -CH-C≡CH.

4. Procédé selon l'une quelconque des revendications 1 à 3, caractérisé en ce que l'on prépare des produits de formule (I) dans laquelle R représente un radical hydrogène ainsi que leurs sels d'addition avec les acides minéraux ou organiques.

5. Procédé selon l'une quelconque des revendications 1 à 3, caractérisé en ce que l'on prépare des produits de formule (I) dans laquelle R représente un radical ainsi que leurs sels d'addition avec les acides minéraux ou organiques.

6. Procédé selon l'une quelconque des revendications 1 à 3, caractérisé en ce que l'on prépare des produits de formule (I) dans laquelle R représente un radical -CO-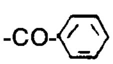 ainsi que leurs sels d'addition avec les acides minéraux ou organiques.

7. Procédé selon l'une quelconque des revendications 1 à 3, caractérisé en ce que l'on prépare des produits de formule (I) dans laquelle R représente un radical -CO-NH-R dans lequel R représente un radical butyle, un radical phényle, un radical 4-méthoxyphényle, un radical 4-chlorophényle ou un radical 4-isopropylphényle ainsi que leurs sels d'addition avec les acides minéraux ou organiques.

8. Procédé selon l'une quelconque des revendications 1 à 7, caractérisé en ce que l'on prépare l'un des produits dont le nom suit :

– l'éther méthylique de (4-chlorophényl) aminocarbonyloxy 1,2,5,6-tétrahydropyridin-3-carboxaldéhyde oxime ainsi que ses sels d'addition avec les acides.

**Revendications pour l'Etat contractant suivant : GR**

1. Procédé pour préparer les composés répondant à la formule (I) :

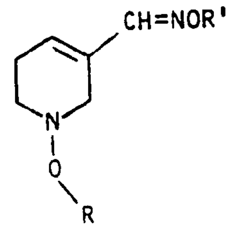

dans laquelle R représente soit un atome d'hydrogène, soit un radical alkyle linéaire, ramifié ou cyclique, saturé ou insaturé renfermant jusqu'à 8 atomes de carbone soit un radical

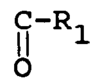

dans lequel R représente un radical alkyle linéaire ou ramifié, saturé ou insaturé renfermant jusqu'à 8 atomes de carbone, un radical alkoxy linéaire ou ramifié, saturé ou insaturé renfermant jusqu'à 8 atomes de carbone, ou un radical aryle renfermant jusqu'à 14 atomes de carbone, soit un radical

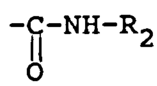

dans lequel R représente un radical alkyle linéaire ou ramifié, saturé ou insaturé renfermant jusqu'à 8 atomes de carbone, un radical aryle renfermant jusqu'à 14 atomes de carbone éventuellement substitué par un ou plusieurs substituants choisis parmi les atomes d'halogène, les radicaux alkyle renfermant jusqu'à 8 atomes de carbone et les radicaux alkoxy renfermant jusqu'à 8 atomes de carbone ou un radical aralkyle renfermant jusqu'à 18 atomes de carbone éventuellement substitué par un ou plusieurs substituants choisis parmi les atomes d'halogène, les radicaux alkyle renfermant jusqu'à 8 atomes de carbone et les radicaux alkoxy renfermant jusqu'à 8 atomes de carbone et R′ représente un radical alkyle linéaire ramifié ou cyclique, saturé ou insaturé renfermant jusqu'à 8 atomes carbone, ainsi que de leurs sels d'addition avec les acides organiques ou minéraux, caractérisé en ce que l'on soumet un composé de formule (II) :

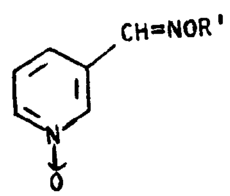

(II)

dans laquelle R′ a la signification indiquée précédemment à l'action d'un agent de réduction pour obtenir le composé de formule ($I_A$) correspondant au produit de formule (I) dans laquelle R représente un atome d'hydrogène

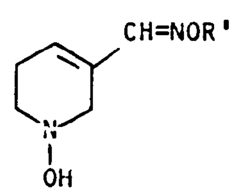

($I_A$)

que l'on soumet si désiré à l'action d'un agent d'estérification, d'éthérification, d'alkoxycarbonylation ou d'amidification pour obtenir le dérivé de formule (I) correspondant que l'on soumet si désiré, à l'action d'un acide pour en former le sel.

2. Procédé selon la revendication 1, caractérisé en ce que l'on utilise au départ, un composé de formule

(II) dans laquelle R′ représente un radical -CH.

3. Procédé selon la revendication 1, caractérisé en ce que l'on utilise au départ, un composé de formule (II) dans laquelle R′ représente un radical -CH-C=CH.

4. Procédé selon l'une quelconque des revendications 1 à 3, caractérisé en ce que l'on prépare des produits de formule (I) dans laquelle R représente un radical hydrogène ainsi que leurs sels d'addition avec les acides minéraux ou organiques.

5. Procédé selon l'une quelconque des revendications 1 à 3, caractérisé en ce que l'on prépare des produits de formule (I) dans laquelle R représente un radical ainsi que leurs sels d'addition avec les acides minéraux ou organiques.

6. Procédé selon l'une quelconque des revendications 1 à 3, caractérisé en ce que l'on prépare des produits de formule (I) dans laquelle R représente un radical -CO-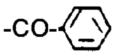 ainsi que leurs sels d'addition avec les acides minéraux ou organiques.

7. Procédé Selon l'une quelconque des revendications 1 à 3, caractérisé en ce que l'on prépare des produits de formule (I) dans laquelle R représente un radical -CO-NH-R dans lequel R représente un radical butyle, un radical phényle, un radical 4-méthoxyphényle, un radical 4-chlorophényle ou un radical 4-isopropylphényle ainsi que leurs sels d'addition avec les acides minéraux ou organiques.

8. Procédé selon l'une quelconque des revendications 1 à 7, caractérisé en ce que l'on prépare l'un des produits dont le nom suit :

– l'éther méthylique de (4-chlorophényl) aminocarbonyloxy 1,2,5,6-tétrahydropyridin-3-carboxaldéhyde oxime ainsi que ses sels d'addition avec les acides.

9. Procédé de préparation de compositions pharmaceutiques, caractérisé en ce que l'on met à titre de principe actif, l'un au moins des produits de formule générale (I), telle que définie à la revendication 1, ou l'un au moins de leurs sels d'addition avec les acides et les bases pharmaceutiquement acceptables, sous une forme destinée à cet usage.

10. Procédé de préparation de compositions pharmaceutiques, caractérisé en ce que l'on met à titre de principe actif, l'un au moins des produits de formule générale (I), telle que définie à l'une quelconque des revendications 2 à 7, ou l'un au moins de leurs sels d'addition avec les acides et les bases pharmaceutiquement acceptables, sous une forme destinée à cet usage.

11. Procédé de préparation de compositions pharmaceutiques, caractérisé en ce que l'on met à titre de principe actif, l'un au moins des produits de formule générale (I), telle que définie à la revendication 1 répondant à la formule suivante :

– l'éther méthylique de (4-chlorophényl) aminocarbonyloxy 1,2,5,6-tétrahydropyridin-3-carboxaldéhyde oxime sous une forme destinée à cet usage.

## Patentansprüche

### Patentansprüche für folgende Vertragsstaaten : AT, BE, CH, DE, FR, GB, IT, LI, LU, NL, SE

1. Verbindungen der allgemeinen Formel (I)

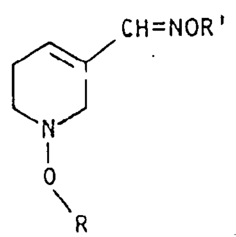

worin R entweder ein Wasserstoffatom oder einen gesättigten oder ungesättigten, linearen, verzweigten oder cyclischen Alkylrest mit bis zu 8 Kohlenstoffatomen oder einen Rest

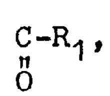

worin R einen gesättigten oder ungesättigten, linearen oder verzweigten Alkylrest mit bis zu 8 Kohlenstoffatomen, einen gesättigten oder ungesättigten, linearen oder verzweigten Alkoxyrest mit bis zu 8 Kohlenstoffatomen oder einen Arylrest mit bis zu 14 Kohlenstoffatomen bedeutet, oder einen Rest

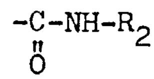

wiedergibt, worin R für einen gesättigten oder ungesättigten, linearen oder verzweigten Alkylrest mit bis zu 8 Kohlenstoffatomen, einen Alkylrest mit bis zu 14 Kohlenstoffatomen, gegebenenfalls substituiert durch einen oder mehrere Substituenten, ausgewählt unter den Halogenatomen, den Alkylresten mit bis zu 8 Kohlenstoffatomen und den Alkoxyresten mit bis zu 8 Kohlenstoffatomen, oder einem Aralkylrest mit bis zu 18 Kohlenstoffatomen, gegebenenfalls substituiert durch einen oder mehrere Substituenten, ausgewählt unter den Halogenatomen, den Alkylresten mit bis zu 8 Kohlenstoffatomen und den Alkoxyresten mit bis zu 8 Kohlenstoffatomen, steht und R′ einen gesättigten oder ungesättigten, linearen, verzweigten oder cyclischen Alkylrest mit bis zu 8 Kohlenstoffatomen bedeutet, sowie ihre Additionssalze mit organischen oder Mineralsäuren.

2. Verbindungen der Formel (I) gemäß Anspruch 1, worin R für ein Wasserstoffatom steht, sowie deren Additionssalze mit organischen oder Mineralsäuren.

3. Verbindungen der Formel (I), wie in Anspruch 1 definiert, worin R einen Rest -COCH bedeutet, sowie deren Additionssalze mit organischen oder Mineralsäuren.

4. Verbindungen der Formel (I) gemäß Anspruch 1, worin R einen Rest -CO- 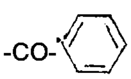 bedeutet, sowie deren Additionssalze mit organischen oder Mineralsäuren,

5. Verbindungen der Formel (I) gemäß Anspruch 1, worin R einen Rest -CONH-R bedeutet, in dem R einen Butylrest, einen Phenylrest, ein 4-Methoxyphenylrest, einen 4-Chlorphenylrest oder einen 4-Isopropylphenylrest bedeutet.

6. Verbindungen der Formel (I) gemäß einem der Ansprüche 1 bis 5, worin R′ für einen Rest -CH steht, sowie deren Additionssalze mit organischen oder Mineralsäuren.

7. Verbindungen der Formel (I) gemäß einem der Ansprüche 1 bis 5, worin R′ für einen Rest -CHC=CH steht, sowie deren Additionssalze mit organischen oder Mineralsäuren.

8. Verbindungen der Formel (I), wie in Anspruch 1 definiert, mit der folgenden Bezeichnung:

– Methylether von (4-Chlorphenyl)-aminocarbonyloxy-1,2,5,6-tetrahydropyridin-3-carboxaldehyd-oxim sowie dessen Additionssalze mit Säuren.

9. Als Arzneimittel die Verbindungen der Formel (I), wie in einem der Ansprüche 1 bis 7 definiert, sowie deren Additionssalze mit pharmazeutisch verträglichen Säuren.

10. Als Arzneimittel die Verbindungen der Formel (I), wie in Anspruch 8 definiert, sowie deren Additionssalze mit pharmazeutisch verträglichen Säuren.

11. Pharmazeutische Zusammensetzungen, enthaltend als Wirkstoff zumindest eines der Arzneimittel gemäß Anspruch 9 oder 10.

12. Verfahren zur Herstellung der Verbindungen der Formel (I), wie in einem der Ansprüche 1 bis 8 definiert, dadurch gekennzeichnet, daß man eine Verbindung der Formel (II)

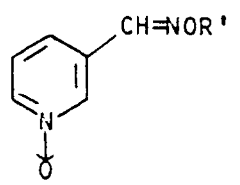

(II)

worin R′ die vorstehend angegebene Bedeutung besitzt, der Einwirkung eines Reduktionsmittels unterzieht, um zu der dem Produkt der Formel (I), worin R für ein Wasserstoffatom steht, entsprechenden Verbindung der Formel ($I_A$)

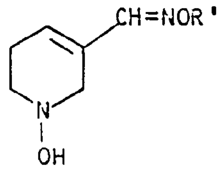

$(I_A)$

zu gelangen, die man gewünschtenfalls der Einwirkung eines Veresterungs-, Veretherungs-, Alkoxycarbonylierungs-oder Amidifizierungsmittels unterzieht, um das entsprechende Derivat der Formel (I) zu erhalten, das man gewünschtenfalls der Einwirkung einer Säure unterzieht, um hieraus das Salz zu bilden.

**Patentansprüche für folgenden Vertragsstaat : ES**

1. Verfahren zur Herstellung der Verbindungen der Formel (I)

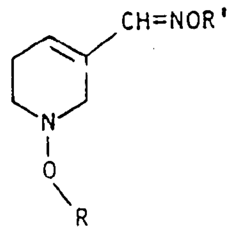

worin R entweder ein Wasserstoffatom oder einen gesättigten oder ungesättigten, linearen, verzweigten oder cyclischen Alkylrest mit bis zu 8 Kohlenstoffatomen oder einen Rest

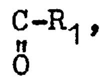

worin R einen gesättigten oder ungesättigten, linearen oder verzweigten Alkylrest mit bis zu 8 Kohlenstoffatomen, einen gesättigten oder ungesättigten, linearen oder verzweigten Alkoxyrest mit bis zu 8 Kohlenstoffatomen oder einen Akylrest mit bis zu 14 Kohlenstoffatomen bedeutet, oder einen Rest

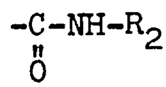

wiedergibt, worin R für einen gesättigten oder ungesättigten, linearen oder verzweiflen Alkylrest mit bis zu 8 Kohlenstoffatomen, einen Alkylrest mit bis zu 14 Kohlenstoffatomen, gegebenefalls substituiert durch einen oder mehrere Substituenten, ausgewählt unter den Halogenatomen, den Alkylresten mit bis zu 8 Kohlenstoffatomen und den Alkoxyresten mit bis zu 8 Kohlenstoffatomen, oder einem Aralkylrest mit bis zu 18 Kohlenstoffatomen, gegebenenfalls substituiert durch einen oder mehrere Substituenten, ausgewählt unter den Halogenatomen, den Alkylresten mit bis zu 8 Kohlenstoffatomen und den Alkoxyresten mit bis zu 8 Kohlenstoffatomen, steht und R′ einen gesättigten oder ungesättigten, linearen, verzweigten oder cyclischen Alkylrest mit bis zu 8 Kohlenstoffatomen bedeutet, sowie ihrer Additionssalze mit organischen oder Mineralsäuren, dadurch **gekennzeichnet** , daß man eine Verbindung der Formel (II)

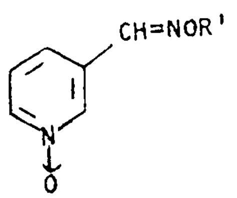

(II)

worin R′ die vorstehend angegebene Bedeutung besitzt, der Einwirkung eines Reduktionsmittels unterzieht,

um zu der dem Produkt der Formel (I), worin R für ein Wasserstoffatom steht, entsprechenden Verbindung der Formel ($I_A$)

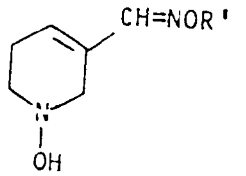

($I_A$)

zu gelangen, die man gewünschtenfalls der Einwirkung eines Veresterungs-, Veretherungs-, Alkoxycarbonylierungsoder Amidifizierungsmittels unterzieht, um das entsprechende Derivat der Formel (I) zu erhalten, das man gewünschtenfalls der Einwirkung einer Säure unterzieht, um hieraus das Salz zu bilden.

2. Verfahren gemäß Anspruch 1, dadurch gekennzeichnet, daß man von einer Verbindung der Formel (II) ausgeht, worin R′ für einen Rest -CH steht.

3. Verfahren gemäß Anspruch 1, dadurch gekennzeichnet, daß man von einer Verbindung der Formel (II) ausgeht, worin R′ für einen Rest -CH-C=CH steht.

4. Verfahren gemäß einem der Ansprüche 1 bis 3, dadurch gekennzeichnet, daß man Produkte der Formel (I), worin R ein Wasserstoffatom bedeutet, sowie deren Additionssalze mit Mineral- oder organischen Säuren herstellt.

5. Verfahren gemäß einem der Ansprüche 1 bis 3, dadurch gekennzeichnet, daß man Produkte der Formel (I), worin R einen Rest -COCH bedeutet, sowie deren Additionssalze mit Mineral- oder organischen Säuren herstellt.

6. Verfahren gemäß einem der Ansprüche 1 bis 3, dadurch gekennzeichnet, daß man Produkte der Formel (I), worin R einen Rest -CO- 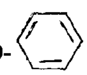 darstellt, sowie deren Additionssalze mit Mineral- oder organischen Säuren herstellt.

7. Verfahren gemäß einem der Ansprüche 1 bis 3, dadurch gekennzeichnet, daß man Produkte der Formel (I), worin R einen Rest -CO-NH-R darstellt, worin R für einen Butylrest, einen Phenylrest, einen 4-Methoxyphenylrest, einen 4-Chlorphenylrest oder einen 4-Isopropylphenylrest steht, sowie deren Additionssalze mit Mineral-oder organischen Säuren herstellt.

8. Verfahren gemäß einem der Ansprüche 1 bis 7, dadurch gekennzeichnet, daß man eines der Produkte mit der folgenden Bezeichnung herstellt:

– Methylether von (4-Chlorphenyl)-aminocarbonyloxy-1,2,5,6-tetrahydropyridin-3-carboxaldehyd-oxim sowie dessen Additionssalze mit Säuren.

**Patentansprüche für folgenden Vertragsstaat : GR**

1. Verfahren zur Herstellung der Verbindungen der Formel (I)

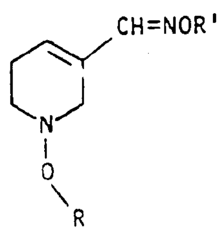

worin R entweder ein Wasserstoffatom oder einen gesättigten oder ungesättigten, linearen, verzweigten oder cyclischen Alkylrest mit bis zu 8 Kohlenstoffatomen oder einen Rest

$$\underset{\overset{\|}{O}}{C}-R_1 ,$$

worin R einen gesättigten oder ungesättigten, linearen oder verzweigten Alkylrest mit bis zu 8 Kohlenstoffatomen, einen gesättigten oder ungesättigten, linearen oder verzweigten Alkoxyrest mit bis zu 8 Kohlenstoffatomen oder einen Arylrest mit bis zu 14 Kohlenstoffatomen bedeutet, oder einen Rest

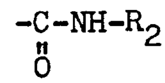

wiedergibt, worin R für einen gesättigten oder gesättigten, linearen oder verzweigten Alkylrest mit bis zu 8 Kohlenstoffatomen, einen Arylrest mit bis zu 14 Kohlenstoffatomen, gegebenefalls substituiert durch einen oder mehrere Substituenten, ausgewählt unter den Halogenatomen, den Alkylresten mit bis zu 8 Kohlenstoffatomen und den Alkoxyresten mit bis zu 8 Kohlenstoffatomen, oder einem Aralkylrest mit bis zu 18 Kohlenstoffatomen, gegebenenfalls substituiert durch einen oder mehrere Substituenten, ausgewählt unter den Halogenatomen, den Aldylresten mit bis zu 8 Kohlenstoffatomen und den Alkoxyresten mit bis zu 8 Kohlenstoffatomen, steht und R′ einen gesättigten oder ungesättigten, linearen, verzweigten oder cyclischen Alkylrest mit bis zu 8 Kohlenstoffatomen bedeutet, sowie ihrer Additionssalze mit organischen oder Mineralsäuren, dadurch **gekennzeichnet** , daß man eine Verbindung der Formel (II)

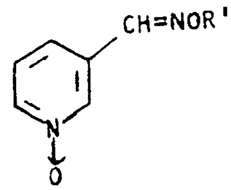

(II)

worin R′ die vorstehend angegebene Bedeutung besitzt, der Einwirkung eines Reduktionsmittels unterzieht, um zu der dem Produkt der Formel (I), worin R für ein Wasserstoffatom steht, entsprechenden Verbindung der Formel ($I_A$)

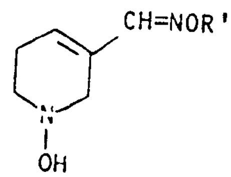

($I_A$)

zu gelangen, die man gewünschtenfalls der Einwirkung eines Veresterungs-, Veretherungs-, Alkoxycarbonylierungsoder Amidifizierungsmittels unterzieht, um das entsprechende Derivat der Formel (I) zu erhalten, das man gewünschtenfalls der Einwirkung einer Säure unterzieht, um hieraus das Salz zu bilden.

2. Verfahren gemäß Anspruch 1, dadurch gekennzeichnet, daß man von einer Verbindung der Formel (II) ausgeht, worin R′ für einen Rest -CH steht.

3. Verfahren gemäß Anspruch 1, dadurch gekennzeichnet, daß man von einer Verbindung der Formel (II) ausgeht, worin R′ für einen Rest -CH-C=CH steht.

4. Verfahren gemäß einem der Ansprüche 1 bis 3, dadurch gekennzeichnet, daß man Produkte der Formel (I), worin R ein Wasserstoffatom bedeutet, sowie deren Additionssalze mit Mineral- oder organischen Säuren herstellt.

5. Verfahren gemäß einem der Ansprüche 1 bis 3, dadurch gekennzeichnet, daß man Produkte der Formel (I), worin R einen Rest -COCH bedeutet, sowie deren Additionssalze mit Mineral- oder organischen Säuren herstellt.

6. Verfahren gemäß einem der Ansprüche 1 bis 3, dadurch gekennzeichnet, daß man Produkte der Formel (I), worin R einen Rest -CO-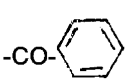 darstellt, sowie deren Additionssalze mit Mineral- oder organischen Säuren herstellt,

7. Verfahren gemäß einem der Ansprüche 1 bis 3, dadurch gekennzeichnet, daß man Produkte der Formel (I), worin R einen Rest -CO-NH-R darstellt, worin R für einen Butylrest, einen Phenylrest, einen 4-Methoxyphenylrest, einen 4-Chlorophenylrest oder einen 4-Isopropylphenylrest steht, sowie deren Additionssalze mit

Mineral-oder organischen Saüren herstellt,

8. Verfahren gemäß einem der Ansprüche 1 bis 7, dadurch gekennzeichnet, daß man eines der Produkte mit der folgenden Bezeichnung herstellt:

– Methylether von (4-Chlorphenyl)-aminocarbonyloxy-1,2,5,6-tetrahydropyridin-3-carboxaldehyd-oxim sowie dessen Additionssalze mit Säuren.

9. Verfahren zur Herstellung von pharmazeutischen Zusammensetzungen, dadurch gekennzeichnet, daß man als Wirkstoff zumindest eines der Produkte der allgemeinen Formel (I), wie in Anspruch 1 definiert, oder zumindest eines ihrer Additionssalze mit pharmazeutisch verträglichen Säuren und Basen in eine für diese Verwendung vorgesehene Form bringt.

10. Verfahren zur Herstellung von pharmazeutischen Zusammensetzungen, dadurch gekennzeichnet, daß man als Wirkstoff zumindest eines der Produkte der allgemeinen Formel (I), wie in einem der Ansprüche 2 bis 7 definiert, oder zumindest eines ihrer Additionssalze mit pharmazeutisch verträglichen Säuren und Basen in eine für diese Verwendung bestimmte Form bringt.

11. Verfahren zur Herstellung von pharmazeutischen Zusammensetzungen, dadurch gekennzeichnet, daß man als Wirkstoff zumindest eines der Produkte der allgemeinen Formel (I), wie in Anspruch 1 definiert, der folgenden Formel

– Methylether von (4-Chlorphenyl)-aminocarbonyloxy-1,2,5,6-tetrahydropyridin-3-carboxaldehyd-oxim in eine für diese Verwendung bestimmte Form bringt.

## Claims

**Claims for the following Contracting States: AT, BE, CH, DE, FR, GB, IT, LI, LU, NL, SE**

1. The compounds corresponding to formula (I):

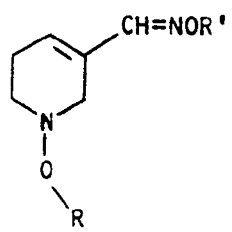

in which R represents either a hydrogen atom, or a saturated or unsaturated, linear, branched or cyclic alkyl radical containing up to 8 carbon atoms or a

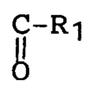

radical in which R represents a saturated or unsaturated, linear or branched alkyl radical containing up to 8 carbon atoms, a saturated or unsaturated, linear or branched alkoxy radical containing up to 8 carbon atoms, or an aryl radical containing up to 14 carbon atoms, or a

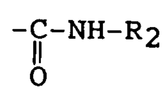

radical in which R represents a saturated or unsaturated, linear or branched alkyl radical containing up to 8 carbon atoms, an aryl radical containing up to 14 carbon atoms optionally substituted by one or more substituents chosen from halogen atoms, alkyl radicals containing up to 8 carbon atoms and alkoxy radicals containing up to 8 carbon atoms or an aralkyl radical containing up to 18 carbon atoms optionally substituted by one or more substituents chosen from halogen atoms, alkyl radicals containing up to 8 carbon atoms and alkoxy radicals containing up to 8 carbon atoms and R′ represents a saturated or unsaturated, linear, branched or cyclic alkyl radical containing up to 8 carbon atoms, as well as their addition salts with organic or mineral acids.

2. The compounds of formula (I) as defined in claim 1 in which R represents a hydrogen atom, as well as

their addition salts with organic or mineral acids.

3. The compounds of formula (I) as defined in claim 1 in which R represents a -COCH radical, as well as their addition salts with organic or mineral acids.

4. The compounds of formula (I) as defined in claim 1 in which R represents a -CO- 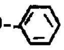 radical, as well as their addition salts with organic or mineral acids.

5. The compounds of formula (I) as defined in claim 1 in which R represents a -CONH-R radical in which R represents a butyl radical, a phenyl radical, a 4-methoxyphenyl radical, a 4-chlorophenyl radical or a 4-isopropylphenyl radical.

6. The compounds of formula (I) as defined in any one of claims 1 to 5 in which R′ represents a -CH radical, as well as their addition salts with organic or mineral acids.

7. The compounds of formula (I) as defined in any one of claims 1 to 5 in which R′ represents a -CHC=CH radical, as well as their addition salts with organic or mineral acids.

8. The compounds of formula (I) as defined in claim 1 of which the name follows:

– (4-chlorophenyl)-aminocarbonyloxy-1,2,5,6-tetrahydropyridin-3-carboxaldehyde oxime methyl ether, as well as its addition salts with acids.

9. As medicaments, the compounds of formula (I) as defined in any one of claims 1 to 7, as well as their addition salts with pharmaceutically acceptable acids.

10. As medicaments, the compounds of formula (I) as defined in claim 8, as well as their addition salts with pharmaceutically acceptable acids.

11. The pharmaceutical compositions containing as active ingredient at least one of the medicaments defined in claim 9 or 10.

12. Preparation process for compounds of formula (I) as defined in any one of claims 1 to 8, characterized in that a compound of formula (II):

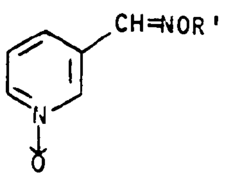

(II)

in which R′ has the meaning indicated previously, is subjected to the action of a reduction agent in order to obtain the compound of formula ($I_A$) corresponding to the product of formula (I) in which R represents a hydrogen atom

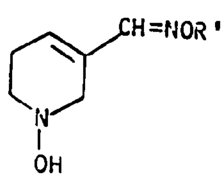

($I_A$)

which is subjected if desired to the action of an esterification, etherification, alkoxycarbonylation or amidification agent in order to obtain the corresponding derivative of formula (I) which is subjected, if desired, to the action of an acid in order to form the salt.

**Claims for the following Contracting State: ES**

1. Process for preparing the compounds corresponding to formula (I):

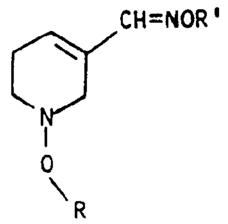

in which R represents either a hydrogen atom, or a saturated or unsaturated, linear, branched or cyclic alkyl radical containing up to 8 carbon atoms or a

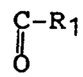

radical in which R represents a saturated or unsaturated, linear or branched alkyl radical containing up to 8 carbon atoms, a saturated or unsaturated, linear or branched alkoxy radical containing up to 8 carbon atoms, or an aryl radical containing up to 14 carbon atoms, or a

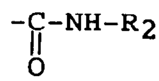

radical in which R represents a saturated or unsaturated, linear or branched alkyl radical containing up to 8 carbon atoms, an aryl radical containing up to 14 carbon atoms optionally substituted by one or more substituents chosen from halogen atoms, alkyl radicals containing up to 8 carbon atoms and alkoxy radicals containing up to 8 carbon atoms or an aralkyl radical containing up to 18 carbon atoms optionally substituted by one or more substituents chosen from halogen atoms, alkyl radicals containing up to 8 carbon atoms and alkoxy radicals containing up to 8 carbon atoms and R′ represents a saturated or unsaturated, linear, branched or cyclic alkyl radical containing up to 8 carbon atoms, as well as their addition salts with organic or mineral acids, characterized in that a compound of formula (II):

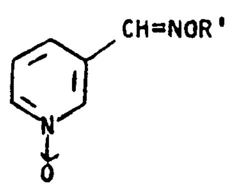

(II)

in which R′ has the meaning indicated previously, is subjected to the action of a reduction agent in order to obtain the compound of formula ($I_A$) corresponding to the product of formula (I) in which R represents a hydrogen atom

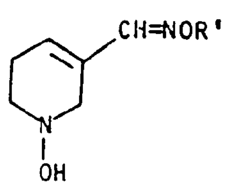

($I_A$)

which is subjected, if desired, to the action of an esterification, etherification, alkoxycarbonylation or amidification agent in order to obtain the corresponding derivative of formula (I) which is subjected, if desired, to the action of an acid in order to form the salt.

2. Process according to claim 1, characterized in that a compound of formula (II) in which R′ represents a -CH radical is used at the start.

3. Process according to claim 1, characterized in that a compound of formula (II) in which R′ represents a -CH-C=CH radical is used at the start.

4. Process according to any one of claims 1 to 3, characterized in that products of formula (I) are prepared in which R represents a hydrogen radical, as well as their addition salts with mineral or organic acids.

5. Process according to any one of claims 1 to 3, characterized in that products of formula (I) are prepared in which R represents a radical, as well as their addition salts with mineral or organic acids.

6. Process according to any one of claims 1 to 3, characterized in that products of formula (I) are prepared in which R represents a -CO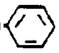 radical, as well as their addition salts with mineral or organic acids.

7. Process according to any one of claims 1 to 3, characterized in that products of formula (I) are prepared in which R represents a -CO-NH-R radical in which R represents a butyl radical, a phenyl radical, a 4-methoxyphenyl radical, a 4-chlorophenyl radical or a 4-isopropylphenyl radical, as well as their addition salts with mineral or organic acids.

8. Process according to any one of claims 1 to 7, characterized in that one of the products is prepared of which the name follows:

– (4-chlorophenyl)-aminocarbonyloxy-1,2,5,6-tetrahydropyridin-3-carboxaldehyde oxime methyl ether, as well as its addition salts with acids.

**Claims for the following Contracting State : GR**

1. Process for preparing the compounds corresponding to formula (I):

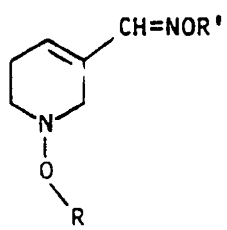

in which R represents either a hydrogen atom, or a saturated or unsaturated, linear, branched or cyclic alkyl radical containing up to 8 carbon atoms or a

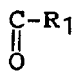

radical in which R represents a saturated or unsaturated, linear or branched alkyl radical containing up to 8 carbon atoms, a saturated or unsaturated, linear or branched alkoxy radical containing up to 8 carbon atoms, or an aryl radical containing up to 14 carbon atoms, or a

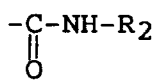

radical in which R represents a saturated or unsaturated, linear or branched alkyl radical containing up to 8 carbon atoms, an aryl radical containing up to 14 carbon atoms optionally substituted by one or more substituents chosen from halogen atoms, alkyl radicals containing up to 8 carbon atoms and alkoxy radicals containing up to 8 carbon atoms or an aralkyl radical containing up to 18 carbon atoms optionally substituted by one or more substituents chosen from halogen atoms, alkyl radicals containing up to 8 carbon atoms and alkoxy radicals containing up to 8 carbon atoms and R′ represents a saturated or unsaturated, linear, branched or cyclic alkyl radical containing up to 8 carbon atoms, as well as their addition salts with organic or mineral acids, characterized in that a compound of formula (II):

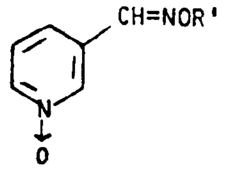

(II)

in which R′ has the meaning indicated previously, is subjected to the action of a reduction agent in order to obtain the compound of formula ($I_A$) corresponding to the product of formula (I) in which R represents a hydrogen atom

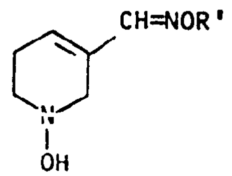

($I_A$)

which is subjected, if desired, to the action of an esterification, etherification, alkoxycarbonylation or amidification agent in order to obtain the corresponding derivative of formula (I) which is subjected, if desired, to the action of an acid in order to form the salt.

2. Process according to claim 1, characterized in that a compound of formula (II) in which R′ represents a -CH radical is used at the start.

3. Process according to claim 1, characterized in that a compound of formula (II) in which R′ represents a -CH-C=CH radical is used at the start.

4. Process according to any one of claims 1 to 3, characterized in that products of formula (I) are prepared in which R represents a hydrogen radical, as well as their addition salts with mineral or organic acids.

5. Process according to any one of claims 1 to 3, characterized in that products of formula (I) are prepared in which R represents a radical, as well as their addition salts with mineral or organic acids.

6. Process according to any one of claims 1 to 3, characterized in that products of formula (I) are prepared in which R represents a -CO-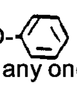 radical, as well as their addition salts with mineral or organic acids.

7. Process according to any one of claims 1 to 3, characterized in that products of formula (I) are prepared in which R represents a -CO-NH-R radical in which R represents a butyl radical, a phenyl radical, a 4-methoxyphenyl radical, a 4-chlorophenyl radical or a 4-isopropylphenyl radical, as well as their addition salts with mineral or organic acids.

8. Process according to any one of claims 1 to 7, characterized in that one of the products is prepared of which the name follows:

– (4-chlorophenyl)-aminocarbonyloxy-1,2,5,6-tetrahydropyridin-3-carboxaldehyde oxime methyl ether, as well as its addition salts with acids.

9. Preparation process for pharmaceutical compositions, characterized in that at least one of the products of general formula (I), as defined in claim 1, or at least one of their addition salts with pharmaceutically acceptable acids and bases, is used as active ingredient in a form intended for this use.

10. Preparation process for pharmaceutical compositions, characterized in that at least one of the products of general formula (I), as defined in any one of claims 2 to 7, or at least one of their addition salts with pharmaceutically acceptable acids and bases, is used as active ingredient in a form intended for this use.

11. Preparation process for pharmaceutical compositions, characterized in that at least one of the products of general formula (I), as defined in claim 1, corresponding to the following formula:

– (4-chlorophenyl)-aminocarbonyloxy-1,2,5,6-tetrahydropyridin-3-carboxaldehyde oxime methyl ether, is used as active ingredient in a form intended for this use.